# EUROPÄISCHE PATENTSCHRIFT

(11) **EP 1 001 732 B1**
(45) Veröffentlichungstag und Bekanntmachung des Hinweises auf die Patenterteilung: **02.04.2003**
(21) Anmeldenummer: 98947328.5
(22) Anmeldetag: 21.07.1998
(51) Int. Cl.: A61K 7/00

(54) **SCHELLACKHALTIGES KOSMETISCHES PRODUKT**
SHELLAC-CONTAINING COSMETIC PRODUCT
PRODUIT COSMETIQUE CONTENANT DE LA SHELLAC

(30) Priorität: 01.08.1997 DE 19734544
(43) Veröffentlichungstag der Anmeldung: 24.05.2000
(73) Patentinhaber: LANCASTER GROUP GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE9802086
(87) Internationale Veröffentlichungsnummer: WO99006011

(56) Entgegenhaltungen:
- EP-A- 0 469 232
- EP-A- 0 615 741
- WO-A-96/14835
- US-A- 5 098 472
- US-A- 5 310 547
- US-A- 5 614 200

## Beschreibung

Die Erfindung betrifft eine kosmetische Emulsion oder ein Gel mit einem Gehalt an Schellack.

Es ist bekannt, daß in kosmetischen Haarsprays, Haarfestigern, Shampoos sowie auch in Nagellacken als Harzkomponente Schellack eingesetzt wurde. Dabei lag der Schellack als filmbildendes Mittel in alkoholischen Zusammensetzungen vor. Schellack wurde weitgehend abgelöst durch eine Reihe moderner Copolymerer, die diese Aufgabe besser übernahmen.

Dennoch ist Schellack nach wie vor für bestimmte Anwendungen wie Haarsprays oder Shampoos verwendet worden, nicht zuletzt, weil es als sehr reines Naturprodukt in guter Qualität verfügbar ist.

Aus der US-A-5614200 sind als Mascara verwendete W/O-Emulsionen bekannt, die Feststoffe, u.a. Harze wie Schellack, flüssige Vehikel, wie Alkohol, und einen Erhärtungsverzögerer neben anderen kosmetischen Hilfssstoffen enthalten.

Weiterhin ist aus der WO96/41630 bekannt, in einem aus drei oder mehr Schichten gebildeten Lichtschutzmittel Schellack als Trennmittel zwischen den voneinander abgesetzten Schichten zu verwenden, so daß sich ein Mehrschichtaufbau des Lichtschutzmittels auf der Haut ausbildet, und die Haut nur mit der Schicht in Kontakt kommt, die hautverträgliche anorganische Lichtschutzmittel enthält.

Der Erfindung liegt die Aufgabe zugrunde, das Hautgefühl (feeling) bei herkömmlichen Emulsionen und Gelen durch Vermeidung eines öligen oder klebrigen Gefühles zu verbessern bei gleichzeitiger Verbesserung der Wasserfestigkeit und der Erzielung stabiler Emulsionen und Gele.

Es wurde nun gefunden, daß ein Zusatz einer wäßrigen Schellackdispersion bei O/W- oder W/O-Emulsionen oder bei Hydrogelen unter bestimmten Bedingungen zu einem kosmetischen Produkt (Emulsion oder Gel) führt, das einen andersartigen Aufbau und damit auch andere Eigenschaften hat.

Voraussetzung für die Herstellung derartiger Emulsionen und Gele ist der Einsatz wäßriger, reiner Schellacklösungen oder - dispersionen. Schellack ist normalerweise nur in Lösungsmitteln wie Alkoholen, Ketonen und organischen Säuren löslich. In Kohlenwasserstoffen ist Schellack unlöslich, in Wasser nur in Form von verseiftem Schellack. Es wurde gefunden, daß auch der unverseifte Schellack unter bestimmten Bedingungen in Wasser bis zu etwa 30 Gew-% löslich ist, und Dispersionen mit Wasser bis zu etwa 60 Gew-% möglich sind. Derartige Lösungen/ Dispersionen bestehen aus Schellack im Bereich von 1 bis 60 Gew-%, vorzugsweise 10 bis 60 Gew-%, insbesondere 15-60 Gew-%;
einem wasserlöslichen Filmbildner im Bereich von 0,1 bis 3 Gew-%;
einem im sauren Bereich beständigen Gelbildner im Bereich von 0,1 bis 1 Gew-%;
einer Säure zur Einstellung des pH-Wertes auf einen Wert von 2 - 4,2, vorzugsweise 2 bis 3,5;
und Wasser bis 100 Gew-%.

Sie werden hergestellt, indem fester, gereinigter, entwachster Schellack unter Rühren bei einer Temperatur im Bereich von 5 bis 20 °C in eine saure wäßrige Lösung des Filmbildners und des Gelbildners bei pH 2 - 4,2 eingetragen und die Bestandteile der Lösung oder Dispersion gut miteinander homogenisiert werden.

Erfindungsgemäß bereitgestellt wird somit ein schellackhaltiges kosmetisches Produkt, gekennzeichnet durch einen Gehalt an reinem Schellack in der wäßrigen Phase, wobei der Schellackanteil
in einer O/W-Emulsion im Bereich von 0,1 bis 20 Gew-%,
in einer W/O-Emulsion im Bereich von 0,1 bis 15 Gew-%,
in einem Hydrogel im Bereich von 0,1 bis 10 Gew-%
liegt, die wäßrige Phase weiterhin einen wasserlöslichen Filmbildner und einen im sauren Bereich bestandigen Gelbildner enthält, und die ölige Phase oder die wäßrige Phase oder beide Phasen weitere kosmetisch wirksame Bestandteile oder Trägerstoffe im Bereich von 1 bis 20 Gew-% enthalten können. Dabei sind alle Angaben auf das Gewicht der Gesamtzusammensetzung bezogen.

Der Schellack rührt aus einer wäßrigen Schellacklösung oder -dispersion her, die aus 5 bis 60 % Schellack, 0,1 bis 3 Gew.-% des Filmbildners, 0,1 bis 1 Gew.-% des Gelbildners, einer Säure zur Einstellung auf pH 2-4,2 und Wasser besteht, bezogen auf die Gesamtkonzentration der Schellacklösung.

Das neue kosmetische Produkt gestattet eine besonders glatte Verteilung auf der Haut ohne ölig zu wirken, erzeugt ein sehr angenehmes weiches Hautgefühl (feeling) und weist keinerlei Klebrigkeit (sticky) auf.

Ein weiterer Gegenstand der Erfindung ist das obige schellackhaltige kosmetische Produkt, gekennzeichnet durch einen Gehalt an reinem Schellack in der wäßrigen Phase, wobei der Schellackanteil
in einer O/W-Emulsion im Bereich von 0,1 bis 20 Gew-%,
in einer W/O-Emulsion im Bereich von 0,1 bis 15 Gew-%,
in einem Hydrogel im Bereich von 0,1 bis 10 Gew-%
liegt,
und die ölige Phase oder die wäßrige Phase oder beide Phasen weitere kosmetisch wirksame Bestandteile oder Trägerstoffe im Bereich von 1 bis 20 Gew-% enthalten können,
erhältlich
a) durch Herstellung einer Emulsion oder eines Hydrogels in üblicher Weise durch Mischen einer primären Emulsionsphase, die darin vorgesehene kosmetische Wirk- und Hilfsstoffe enthält, mit einer sekundären Emulsionsphase, die darin vorgesehene kosmetische Wirk- und Hilfsstoffe enthält,
   oder Mischen eines Gelbildners mit Wasser und Zugabe vorgesehener kosmetischer Wirk- und Hilfsstoffe;
   und
b) Herstellung einer 5 bis 60 Gew-% wäßrigen Schellacklösung oder -dispersion durch Mischen von reiner Schellack-Trockensubstanz mit einem Molekulargewicht von etwa 1000 bis 1010 mit Wasser in Gegenwart eines wasserlöslichen Filmbildners und eines im sauren pH-Bereich beständigen Gelbildners bei einer Temperatur von 5 bis 20 °C und einem pH-Wert von 2 bis 4,2;
   und
c) Erwärmen der Emulsion oder des Gels von Stufe a) auf eine Temperatur im Bereich von 35 bis 45 °C unter Rühren;
   und
d) Zugabe der Schellacklösung oder -dispersion von Stufe b) dazu unter Rühren bei 150 bis 1000 U/Min während eines Zeitraumes von 5 bis 60 Minuten bei 20 bis 45 °C ; und danach
e) Abkühlen des erhaltenen Produktes auf Umgebungstemperatur.

Die Temperatur für den Verfahrensschritt b) liegt vorteilhaft im Bereich von 5 bis 15 °C.

Als wasserlöslicher Filmbildner kann z.B ein Polyvinylpyrrolidon eingesetzt werden, z.B. PVPK30®. Weitere mögliche Filmbildner sind Chitosan, mikrokristallines Chitosan, quarterniertes Chitosan, Polymere der Acrylsäurerreihe, quarternäre Cellulosederivate, Hyaluronsäure und deren Salze; sowie Gemische davon.

Ein im sauren Bereich beständiger Gelbildner ist z.B ein modifiziertes natürliches Polysaccharid wie Guar Hyroxypropyltrimonium Chloride (Jaguar C14S®). Weitere mögliche Gelbildner sind Agar, Alginate, Alginsäure; sowie Gemische davon.

Bei dem hier eingesetzten Schellack handelt es sich um ein aufbereitetes Produkt. Schellack ist bekanntlich ein natürliches Harz, das von der weiblichen Lackschildlaus Kerria lacca ausgeschieden wird. Das für kosmetische Zwecke eingesetzte, gereinigte, wachsfreie Harz, das ein Molekulargewicht von etwa 1000 hat, besteht aus einer Reihe von Hydroxymonodicarbonsäuren in Form von Lactonen, Lactiden und intramolekularen Estern. Die Hauptkomponenten sind Aleuritinsäure, Schellolsäure und Jalarsäure (C₁₅H₂₀O₅) mit Molgewichten von 304, 296 bzw. 280. Schellack ist daher eher ein Oligomeres von Hydroxycarbonsäuren als ein Polymeres. Mittels eines Lösungsmittelextraktionsverfahrens z.B. mit einem Alkohol, können Verunreinigungen entfernt werden, und nach Entfärbung mit Aktivkohle und Verdampfen des Alkohols kann Schellack in einen gelblichen bis schwach gelblichen Feststoff überführt werden.
Ein solcher Feststoff ist Ausgangsprodukt der wäßrigen Lösungen oder Dispersionen, die bei der Erfindung eingesetzt werden.

Erstmalig wird durch die vorliegende Erfindung der Einsatz von Schellack in reiner Form als wäßrige Lösung oder Dispersion mit Viskositäten von >100 mPa·s (cP) in wäßrigen kosmetischen Produkten mit Vorteil möglich, insbesondere in O/W-Emulsionen.

Die eingesetzten Schellacklösungen/dispersionen haben bei etwa 10 Gew-% Schellackgehalt eine Viskosität von ca. 500 mPa·s (cP), bei 20 Gew-% von ca. 1000 mPa·s (cP) und bei 35 Gew-% von ca. 2500 mPa·s (cP). Bevorzugt sind Schellacklösungen oder - dispersionen von etwa 400 - 500 mPa·s (cP) und höher. So haben Dispersionen von 55 - 60 Gew-% Schellack Viskositäten von ca. 100.000 mPa·s (cP), sind cremig bis pastenartig und lassen sich mit den entsprechenden Apparaturen auch noch gut verarbeiten. Wegen der Abhängigkeit der Viskosität von der Konzentration des Schellack in wäßrigen Dispersionen und Lösungen ist es zweckmäßig, bei der praktischen Umsetzung der Erfindung mit Viskositäten zu arbeiten.

Der Aufbau einer erfindungsgemäßen Emulsion ist bei einer O/W-Emulsion vermutlich so, daß der Schellack verteilt in der kontinuierlichen Wasserphase vorliegt, oder daß in einer W/O-Emulsion Wassertröpfchen umhüllt sind von einer Schicht des natürlichen Harzes Schellack, oder in einem Hydrogel Schellacktröpfchen in statistischer Verteilung in der wäßrigen gelierten Phase vorliegen.

Dabei liegt in der Emulsion oder in dem Hydrogel der Schellackanteil im Bereich von 0,5 bis 20 Gew-%, vorzugsweise im Bereich von 0,5 bis 8 Gew-%.

In der O/W-Emulsion liegt der Ölanteil im Bereich von 3 bis 20 Gew-% liegt, vorzugsweise 3,5 bis 7,7 Gew-%, und der Wasseranteil liegt im Bereich von 50 bis 80 Gew-%, vorzugsweise 50 bis 65 Gew-%.

In der W/O-Emulsion liegt der Wasseranteil im Bereich von 40 bis 65 Gew-%, vorzugsweise 40 bis 55 Gew-%, und der Ölanteil liegt im Bereich von 5 bis 20 Gew-%, vorzugsweise 4,5 bis 10 Gew-%. In dem Hydrogel liegt der Wasseranteil im Bereich von 40 bis 80 Gew-% , vorzugsweise 50 bis 80 Gew-%.

Weiterhin bevorzugt ist, daß die Konzentration der wäßrigen Schellacklösung oder -dispersion 15 bis 50 Gew-% beträgt.

Das erfindungsgemäße kosmetische Produkt kann auch eine Mehrfachemulsion vom Typ W/O/W oder O/W/O sein.

Vorzugsweise liegt der Schellackanteil im fertigen kosmetischen Produkt in einer O/W-Emulsion im Bereich von 5 - 20 Gew-%, in einer W/O-Emulsion im Bereich von 5 - 15 Gew-%, in einem Hydrogel im Bereich von 3 bis 10 Gew-%.

Das schellackhaltige kosmetische Produkt der Erfindung liegt vorteilhaft als Creme, Lotion, Sonnenschutzpräparat, Lippenstift, Make-up, Maske, Lippenglanz, Spray oder Gel vor.

Die ölige Phase oder die wäßrige Phase oder beide Phasen können weitere kosmetisch wirksame Bestandteile und kosmetische Hilf- oder Trägerstoffe enthalten .

Kosmetischen Wirkstoffe sind beispielsweise Emulgatoren, wie 1-Decan,Homopolymer; Polyglycerine Ester und andere bekannte Emulgatoren für W/O-Emulsionen, weiterhin C12-C15-Alkyl-benzoate, Bienenwachs (Beeswax), Steareth, Schibutter (Sheabutter) und andere bekannte O/W-Emulgatoren.

Weitere mögliche Zusatzstoffe sind Isopropylmyristat, Isopropylpalmitat, Isopropylstearat, Carbomer, Cetearylalkohol, Lecithin, Copolymere, Paraffinöl, Cetylalkohol, Propylenglycol, Polyglycol, Jojobaöl, Siliconöl, Kokosnußöl, Palmöl, Mineralöl, Cetylpalmitat, Acrylates C10-C30 Alkyl Acrylate Crosspolymer, Magnesium Aluminium Silicate, Hydroxyethylcellulose sowie weitere, den speziellen Anwendungsformen wie z. B. Lippenstift, Augenkosmetik, Make-up usw. angepaßte Stoffe, die dem Fachmann bekannt sind.

Als weitere Wirkstoffe kann das Präparat enthalten 1.3 und 1.6-β-Glucan, CM-Glucan®, Allantoin, TiO₂, ZnO, ZrO₂, SiO₂ und weitere UVA- und UVB-blockierende Substanzen, wie organische Lichtschutzmittel. Dafür können eingesetzt werden z.B. Benzophenon-Derivate, 3-Benzylidencampher-Derivate, Ester der Zimtsäure oder der Salicylsäure, 4-Aminobenzoesäure-Derivate, wie zum Beispiel Benzophenone-3, Butylmethoxydibenzoylmethane, Octylmethoxycinnamate, Octylsalicylate sowie auch wasserlösliche Produkte wie Phenylbenimidazolsulfonsäure oder Benzophenonsulfonsäure-Derivate. Auch Kombinationen mit UVA-Filtern sind möglich.

Eine erfindungsgemäße Emulsion kann auch pulverförmige substanzen enthalten, die ausgewählt sind aus der Gruppe, bestehend aus Pigmenten wie Metalloxide (Eisenoxide, TiO₂, Ultramarinblau, Chromoxide, Glimmer-Titanoxid, Glimmer-Eisenoxid, Gemischen davon) und aus anderen pulverförmigen Stoffen, wie SiO₂, Silica, ZnO, Kaolin, mit SiO₂ modifiziertem Kaolin (gemäß WO96/17588), Polytetrafluorethylen, Nylon, Talkum, Glimmer, Polymethylmethacrylat, Polyethylen, natürlichen organischen Verbindungen wie verkapselte oder unverkapselte Getreidestärke und Gemischen davon.

Als kosmetisch nützliche Additive können darüber hinaus eingesetzt werden Wasser, ein- oder mehrwertige Alkohole, wie zum Beispiel Ethanol, Isopropanol, Propylenglycol, Glycerin, Ethylenglycol, C₉-C₁₁-Alkohole usw.; Methyl-, Ethyl- oder Butylether mit Ethylenglycol, Propylenglycol oder Diethylenglycol; Gelbildner; Vitamine wie z.B. Vitamin A, E, B₂, B₆, B₁₂, C; Panthenol, Aloe vera, Allantoin, Bisabolol; Farbstoffe; Konservierungsmittel; Schutzmittel; Radikalfänger; Enzyme; pflanzliche Wirkstoffe; entzündungswidrige natürliche Wirkstoffe; Feuchthaltemittel; Antioxidationsmittel; pH-Regulatoren; Pigmente wie lösliches Melanin; Parfüm; Kupfergluconat usw.

Darüber hinaus kann die Emulsion oder das Gel mit Sauerstoff beladene asymmetrische lamellare Aggregate gemäß WO94/00109 oder dispergierte oder in den asymmetrischen lamellaren Aggregaten oder in Liposomen enthaltene hartmagnetische Einbereichsteilchen mit hoher Koerzitiv-Feldstärke gemäß WO95/03061 enthalten. Sehr vorteilhaft sind auch Aufschlußprodukte von Hefen oder pflanzlichen Stoffen, hergestellt durch ein schonendes Ultraschall-Aufschlußverfahren gemäß WO94/13783.

Die Erfindung hat folgende Vorteile:
1. Die Einbeziehung von Schellack in Emulsionen oder Hydrogele führt zu einem verbesserten Hautgefühl, was durch Tests nachgewiesen werden konnte.
2. Für dekorative Kosmetik, also Lippenstifte, getönte Tagescremes, Make up's, Puder sowie alle Produkte mit Pigmenten, auch pigmentartigen Stoffen wie hartmagnetische Einbereichsteilchen aus Barium- oder Strontiumhexaferrit (siehe WO95/03061), wird die Haftfestigkeit auf der Haut erhöht.
3. Bei Sonnenprodukten, also Sonnencremes, Sommenemulsionen, After-sun-Kuren, Sonnengelen usw. wird die Wasserstabilität erhöht.
4. In Sonnenprodukten wirkt es als Anti-Salzwasser/Sand, da keinerlei ölige Filme auf der Haut zurückbleiben.
5. Bei allen Produkten wird keine Klebrigkeit auf der Haut festgestellt.
6. Es werden homogene Emulsionen erhalten auch bei hohen Anteilen von anorganischen Pigmenten, wie TiO₂ oder ZnO oder Kombinationen von TiO₂, ZnO und SiO₂, was insbesondere für Sonnenprodukte mit hohen Lichtschutzfaktoren vorteilhaft ist.
7. Bei Lippenstiften und Lippenglanz wird ein besonders dauerhaftes (long lasting) Produkt erzielt mit einem speziellen Glanz, der auch als matte Brillianz zu bezeichnen ist.

Die Erfindung soll nachstehend durch Beispiele näher erläutert werden. Alle Angaben sind auf das Gewicht bezogen, sofern nichts anderes ausdrücklich angegeben ist.

### Beispiel 1

### Herstellung einer O/W-Emulsion

Es wurde eine O/W-Emulsion aus folgenden Bestandteilen hergestellt. Dabei wurden die Phasen A und B bei 60 °C unter Rühren separat bereitet und bei o.g. Temperatur zusammengeführt und auf 40 °C abgekühlt. Danach erfolgte die Zugabe der Phasen C und D unter Rühren zu dem Gesamtgemisch.

| **Phase A** | |
|---|---|
| C12-15-Alkyl Benzoate | 3,5 % |
| Steareth | 1,5 % |

| **Phase B** | |
|---|---|
| Wasser | ad 100 % |
| Glycerin | 1,5 % |
| Farbpigmente | 2,0 % |

| **Phase D** | |
|---|---|
| Babassu-öl | 2,5 % |
| Siliconöl | 1,5 % |
| Konservierungsmittel | 0,3 % |

| **Phase D** | |
|---|---|
| Parfümöl | 0,2 % |

### Beispiel 2

### Herstellung einer wäßrigen Schellacklösung/dispersion

In 100 ml Wasser wurde unter Rühren der pH-Wert mit Citronensäure auf 3,3 eingestellt. Danach wurde 1,3 g Polyvinylpyrrolidon (PVPK30®) und 0,6 g Guar Hydroxypropyltrimonium Chloride (CTFA-Name) (Jaguar C14S®) unter weiterem Rühren hinzugegeben. In die erhaltene Lösung wurden 15 g gereinigter, entwachster, teilchenförmiger Schellack bei etwa 13-15 °C und bei weniger als 1000 U/Min. eingetragen und die Lösung bei 1450 U/Min. homogenisiert.

Die auf diese Weise hergestellte Schellacklösung hatte eine Viskosität von 540 mPa·s (cP). Unter Variierung des pH-Wertes, der Menge an eingesetztem Schellack und der Mengen der anderen eingesetzten Komponenten wurden in gleicher Weise Lösungen bzw. Dispersionen mit höheren Viskositäten bzw. Konzentrationen an Schellack hergestellt.

### Beispiel 3

### Herstellung einer W/O-Emulsion

Es wurde eine W/O-Emulsion aus folgenden Bestandteilen hergestellt. Dabei wurden die Phasen A und B bei 85 °C unter Rühren separat bereitet und bei o.g. Temperatur zusammengeführt und unter Rühren auf 40 °C abgekühlt. Danach erfolgte die Zugabe der Phase C unter Rühren zu dem Gesamtgemisch.

| **Phase A** | |
|---|---|
| 1-Decan | 11,0 % |
| Polyglycerin Ester | 3,0 % |
| Mineralöl | 5,0 % |
| UV-Filter TiO₂/ZnO/Benzophenone-3 | 3,5 % |
| SiO₂ (monodispers) (Merck) | 1,0 % |

| **Phase B** | |
|---|---|
| Destilliertes Wasser | ad 100 % |
| Glycerin | 6,0 % |

| **Phase C** | |
|---|---|
| Konservierungsmittel | 0,2 % |
| Parfümöl | 0,1 % |

### Beispiel 4

### Herstellung eines Gels

Die Phasen A bis C wurden hergestellt und in dieser Reihenfolge nacheinander bei Raumtemperatur miteinander vermischt und homogenisiert.

| **Phase A** | |
|---|---|
| Destilliertes Wasser | ad 100 % |
| Crosspolymer | 1,0 % |
| Glycerin | 2,0 % |

| **Phase B** | |
|---|---|
| Triethanolamin | 1,0 % |

| **Phase C** | |
|---|---|
| Parfümöl | 0,2 % |
| Konservierungsmittel Kamillenextrakt | 2,0 % |
| Vitamin E | 0,5 % |
| Vitamin C | 0,5 % |
| Vitamin B₂ | 1,0 % |

### Beispiel 5

### Tönende Tagescreme

Eine Schellacklösung mit einem Schellackgehalt von 20 %, hergestellt gemäß Beispiel 2, wurde als Phase E mit einem Anteil von 15,0 % an der Gesamtzusammensetzung bei 35 °C unter Rühren bis zur Homogenität dem Gemisch von Beispiel 1 zugegeben.

### Beispiel 6

### Sonnenemulsion

Eine Schellacklösung mit einem Schellackgehalt von 10 %, hergestellt gemäß Beispiel 2, wurde als Phase D mit einem Anteil von 20,5 % an der Gesamtzusammensetzung bei 35 °C unter Rühren bis zur Homogenität dem Gemisch von Beispiel 3 zugesetzt.

### Beispiel 7

### Pflege-Gel

Eine Schellacklösung oder -dispersion mit einem Schellackgehalt von 1 %, hergestellt gemäß Beispiel 2, wurde als Bestandteil der Phase C mit einem Anteil von 20,5 % an der Gesamtzusammensetzung bei Umgebungstemperatur unter Rühren bis zur Homogenität dem Gemisch von Beispiel 4 zugesetzt.

### Beispiel 8

### Lippenstift

| **Phase A** | |
|---|---|
| Wachs WM300/46 (CTFA-Name) | 70 % |
| Castoröl | 12 % |
| PPG 1-Glyceryl Oleostearate Paraffin | 0,5 % |
| Farbstoffe | 4,5 % |

| **Phase B** | |
|---|---|
| DMDM Hydantoin | 0,6 % |
| PPG 51SMDI Copolymer (CTFA-Name) | 1,0 % |
| Wasser | q.s. |

| **Phase C** | |
|---|---|
| Parfümöl | 0,5 % |

Die Phase A wurde unter Rühren bei 80 °C geschmolzen. Zur Phase B wurde bei 80 °C die Phase A unter Rühren zugegeben. Danach wurde das Gemisch abgekühlt auf 50 °C, und die Phase C bei dieser Temperatur unter Rühren hinzugesetzt. Danach wurde eine eine Schellackdispersion mit einem Schellackgehalt von 50 %, hergestellt gemäß Beispiel 2, mit einem Anteil von 5 % an der Gesamtzusammensetzung unter Rühren bis zur Homogenität zugegeben und das Produkt erstarren lassen.

### Vergleichsbeispiel 1

Es wurde ein Produkt gemäß Beispiel 6 hergestellt, das mit "Produkt A" bezeichnet wurde. Es wurde ein ähnliches Produkt wie im Beispiel 6 hergestellt, bei dem keine Schellacklösung zugegeben wurde. Diese Produkt wurde mit "Produkt B" bezeichnet.

Die beiden Produkte wurden von 10 vorgebräunten Probanden zum Testen während des Sonnenbades auf dem ganzen Körper aufgetragen.

Das Produkt A zeigte einen ausreichenden UV-Schutz bei allen Probanden. Es wurde im Zeitraum des täglichen 4-stündigen Sonnenbades nur jeweils einmal aufgetragen und zeigte eine ausreichende Wasserresistenz bei dreimaligen Unterbrechungen durch ein Wasserbad von jeweils 10 Minuten.

Produkt B wurde von 8 Probanden im Zeitraum des täglichen 4-stündigen Sonnenbades wenigstens zweimal aufgetragen, um den gleichen UV-Schutz zu erreichen. Die Wasserbeständigkeit war nicht ausreichend.

## Patentansprüche

1. Schellackhaltiges kosmetisches Produkt, **gekennzeichnet durch** einen Gehalt an Schellack in der wäßrigen Phase, wobei der Anteil an reinem Schellack in einer O/W-Emulsion im Bereich von 0,1 bis 20 Gew-%, in einer W/O-Emulsion im Bereich von 0,1 bis 15 Gew-%, in einem Hydrogel im Bereich von 0,1 bis 10 Gew-% liegt,
die wäßrige Phase weiterhin einen wasserlöslichen Filmbildner und einen im sauren Bereich beständigen Gelbildner enthält,
und die ölige Phase oder die wäßrige Phase oder beide Phasen weitere kosmetisch wirksame Bestandteile oder Trägerstoffe im Bereich von 1 bis 20 Gew-% enthalten, wobei alle Angaben auf das Gewicht der Gesamtzusammensetzung bezogen sind, und wobei der Schellack aus einer wäßrigen Schellacklösung oder -dispersion herrührt, die aus 5 bis 60 % Schellack, 0,1 bis 3 Gew-% des Filmbildners, 0,1 bis 1 Gew-% des Gelbildners, einer Säure zur Einstellung auf pH 2-4,2 und Wasser besteht, bezogen auf die Gesamtkonzentration der Schellacklösung.

2. Schellackhaltiges kosmetisches Produkt, **gekennzeichnet durch** einen Gehalt an Schellack in der wäßrigen Phase, wobei der Anteil an reinem Schellack
in einer O/W-Emulsion im Bereich von 0,1 bis 20 Gew-%,
in einer W/O-Emulsion im Bereich von 0,1 bis 15 Gew-%,
in einem Hydrogel im Bereich von 0,1 bis 10 Gew-% liegt,
und die ölige Phase oder die wäßrige Phase oder beide Phasen weitere kosmetisch wirksame Bestandteile oder Trägerstoffe im Bereich von 1 bis 20 Gew-% enthalten,
und wobei alle Angaben auf das Gewicht der Gesamtzusammensetzung bezogen sind, erhältlich
a) **durch** Herstellung einer Emulsion oder eines Hydrogels in üblicher Weise **durch** Mischen einer primären Emulsionsphase, die darin vorgesehene kosmetische Wirk- und Hilfsstoffe enthält, mit einer sekundären Emulsionsphase, die darin vorgesehene kosmetische Wirk- und Hilfsstoffe enthält,
oder Mischen eines Gelbildners mit wasser und Zugabe vorgesehener kosmetischer Wirk- und Hilfsstoffe;
und
b) Herstellung einer 5 bis 60 Gew-% wäßrigen Schellacklösung oder -dispersion **durch** Mischen von reiner Schellack-Trockensubstanz mit einem Molekulargewicht von etwa 1000 mit Wasser in Gegenwart von einem wasserlöslichen Filmbildner im Bereich von 0,1 bis 3 Gew-% und einem im sauren Bereich beständigen Gelbildner im Bereich von 0,1 bis 1 Gew-% und einer Säure zur Einstellung des pH-Wertes auf einen Wert von 2 - 4,2 unter Rühren bei 150 bis 1000 U/Min während eines Zeitraumes von 5 bis 60 Minuten bei 5 bis 20 °C;
und
c) Erwärmen der Emulsion oder des Gels von Stufe a) auf eine Temperatur im Bereich von 35 bis 45 °C unter Rühren und
d) Zugabe der Schellacklösung oder -dispersion von Stufe b) unter Rühren bei 150 bis 1000 U/Min während eines Zeitraumes von 5 bis 60 Minuten bei 35 bis 45 °C ;
und danach
e) Abkühlen des erhaltenen Produktes auf Umgebungstemperatur.

3. Produkt nach Anspruch 2, **dadurch gekennzeichnet, daß** die Konzentration der wäßrigen Schellacklösung oder -dipsersion 10 bis 60 Gew-% beträgt.

4. Produkt nach Anspruch 1 oder 2, **dadurch gekennzeichnet, daß** die Konzentration der wäßrigen Schellacklösung oder -dipsersion 15 bis 60 Gew-% beträgt.

5. Produkt nach Anspruch 4, **dadurch gekennzeichnet, daß** die Konzentration der wäßrigen Schellacklösung oder -dipsersion 15 bis 50 Gew-% beträgt.

6. Produkt nach Anspruch 1, **dadurch gekennzeichnet, daß** es eine Mehrfachemulsion vom Typ W/O/W oder O/W/O ist.

7. Produkt nach Anspruch 1 oder 2, **dadurch gekennzeichnet, daß** der Schellackanteil im fertigen kosmetischen Produkt in einer O/W-Emulsion im Bereich von 5 - 20 Gew-%, in einer W/O-Emulsion im Bereich von 5 - 15 Gew-%, in einem Hydrogel im Bereich von 3 bis 10 Gew-% liegt.

8. Produkt nach Anspruch 1 oder 2, **dadurch gekennzeichnet, daß** der Schellackanteil in der Emulsion oder dem Hydrogel im Bereich von 0,5 bis 8 Gew-% liegt.

9. Produkt nach einem der Ansprüche 1 bis 8, **dadurch gekennzeichnet, daß** die Viskosität der Schellacklösung oder -dispersion im Bereich von 500 bis 100.000 mPa·s (cP) liegt.

10. Produkt nach Anspruch 9, **dadurch gekennzeichnet, daß** die Viskosität der Schellacklösung oder -dispersion im Bereich von 1000 bis 100.000 mPa·s (cP) liegt.

11. Produkt nach Anspruch 9, **dadurch gekennzeichnet, daß** die Viskosität der Schellacklösung oder -dispersion im Bereich von 2500 bis 100.000 mPa·s (cP) liegt.

12. Produkt nach einem der Ansprüche 1 bis 11, **dadurch gekennzeichnet, daß** es als Creme, Lotion, Sonnenschutzpräparat, Lippenstift, Make-up, Maske, Lippenglanz, Spray oder Gel vorliegt.

13. Verfahren zur Herstellung eines schellackhaltigen kosmetischen Produktes, **dadurch gekennzeichnet, daß** man die folgenden Schritte durchführt:
a) Herstellung einer Emulsion oder eines Hydrogels in üblicher Weise durch Mischen einer primären Emulsionsphase, die darin vorgesehene kosmetische Wirk- und Hilfsstoffe enthält, mit einer sekundären Emulsionsphase, die darin vorgesehene kosmetische Wirk- und Hilfsstoffe enthält,
oder Mischen eines Gelbildners mit Wasser und Zugabe vorgesehener kosmetischer Wirk- und Hilfsstoffe;
und
b) Herstellung einer 5 bis 60 Gew-% wäßrigen Schellacklösung oder -dispersion durch Mischen von reiner Schellack-Trockensubstanz mit einem Molekulargewicht von etwa 1000 mit Wasser in Gegenwart von einem wasserlöslichen Filmbildner im Bereich von 0,1 bis 3 Gew-% und einem im sauren Bereich beständigen Gelbildner im Bereich von 0,1 bis 1 Gew-% und einer Säure zur Einstellung des pH-Wertes auf einen Wert von 2 - 4,2 unter Rühren bei 150 bis 1000 U/Min während eines Zeitraumes von 5 bis 60 Minuten bei 5 bis 20 °C;
und
c) Erwärmen der Emulsion oder des Gels von Stufe a) auf eine Temperatur im Bereich von 35 bis 45 °C unter Rühren und
d) Zugabe der Schellacklösung oder -dispersion von Stufe b) unter Rühren bei 150 bis 1000 U/Min während eines Zeitraumes von 5 bis 60 Minuten bei 35 bis 45 °C ;
und danach
e) Abkühlen des erhaltenen Produktes auf Umgebungstemperatur.

14. Verfahren nach Anspruch 13, **dadurch gekennzeichnet, daß** die Temperatur im Bereich von 5 bis 15 °C liegt.

15. Verfahren nach Anspruch 13, **dadurch gekennzeichnet, daß** der pH-Wert im Bereich von 2 bis 3,5 liegt.

## Claims

1. A cosmetic product containing shellac, comprising a shellac content in the aqueous phase, where the amount of pure shellac in an O/W emulsion is in a range of 0.1 to 20 wt%, in a W/O emulsion is in a range of 0.1 to 15 wt%, in a hydrogel in the range of 0.1 to 10 wt%; the aqueous phase also contains a water-soluble film-forming substance and a gel-forming substance that is stable in an acid range, and the oily phase or the aqueous phase or both phases contain additional active cosmetic ingredients or vehicles in the range of 1 to 20 wt%, all amounts based on the weight of the total composition, and wherein the shellac results from an aqueous shellac solution or shellac dispersion consisting of 5 to 60 % shellac, 0.1 to 3 wt% of the film-forming substance, 0.1 to 1 wt% of the gel-forming substance, an acid for adjusting the pH to a value of 2 to 4.2, and water, related to the the weight of the total composition.

2. A cosmetic product containing shellac, comprising a shellac content in the aqueous phase, where the amount of pure shellac is
in a range of 0.1 to 20 wt% in an O/W emulsion,
in a range of 0.1 to 15 wt% in a W/O emulsion,
in a range of 0.1 to 10 wt% in a hydrogel,
and the oily phase or the aqueous phase or both phases contain additional active cosmetic ingredients or vehicles in the range of 1 to 20 wt%, where all amounts are based on the weight of the total composition, available by
a) preparing an emulsion or a hydrogel in the usual manner by mixing a primary emulsion phase containing active cosmetic ingredients and additives therein, with a secondary emulsion phase containing active cosmetic ingredients and additives therein,
or mixing a gel-forming agent with water and adding the active cosmetic ingredients and additives provided;
and
b) preparing a 5 to 60 wt% aqueous shellac solution or dispersion by mixing pure shellac dry solids having a molecular weight of approx. 1000 with water in the presence of a water-soluble film-forming agent in the range of 0.1 to 3 wt% and a gel-forming agent that is stable in the acidic range in a concentration range of 0.1 to 1 wt% and an acid for adjusting the pH to a value of 2 to 4.2 while stirring at 150 to 1000 rpm for a period of 5 to 60 minutes at 5 °C to 20 °C;
and
c) heating the emulsion or the gel from step a) to a temperature in the range of 35 °C to 45 °C while stirring and
d) adding the shellac solution or dispersion from step b) while stirring at 150 to 1000 rpm for a period of 5 to 60 minutes at 35 °c to 45 °C;
and then
e) cooling the resulting product to ambient temperature.

3. A product according to claim 2, wherein the concentration of the aqueous shellac solution or dispersion amounts to 10 to 60 wt%.

4. A product according to claim 1 or 2, wherein the concentration of the aqueous shellac solution or dispersion amounts to 15 to 60 wt%.

5. A product according to claim 4, wherein the concentration of the aqueous shellac solution or dispersion amounts to 15 to 50 wt%.

6. A product according to claim 1, comprising a multiple emulsion of the W/O/W type or the O/W/O type.

7. A product according to claim 1 or 2, wherein the shellac content in the finished cosmetic product is in the range of 5-20 wt% in an O/W emulsion, in the range of 5-15 wt% in a W/O emulsion, or in the range of 3-10 wt% in a hydrogel.

8. A product according to claim 1 or 2, wherein the shellac content in the emulsion or the hydrogel is in the range of 0.5 to 8 wt%.

9. A product according to one of claims 1 through 8, wherein the viscosity of the shellac solution or dispersion is in the range of 500 to 100,000 mPa·s (cP).

10. A product according to claim 9, wherein the viscosity of the shellac solution or dispersion is in the range of 1000 to 100,000 mPa·s (cP).

11. A product according to claim 9, wherein the viscosity of the shellac solution or dispersion is in the range of 2500 to 100,000 mPa·s (cP).

12. A product according to one of claims 1 through 11, wherein it is in the form of a cream, lotion, sunscreen preparation, lipstick, makeup, mask, lip gloss, spray or gel.

13. A method of producing a cosmetic product containing shellac, comprising the following steps:
a) preparing an emulsion or a hydrogel in the usual manner by mixing a primary emulsion phase containing active cosmetic ingredients and additives therein, with a secondary emulsion phase containing the active cosmetic ingredients and additives therein,
or mixing a gel-forming agent with water and adding the active cosmetic ingredients and additives provided;
and
b) preparing a 5 to 60 wt% aqueous shellac solution or dispersion by mixing pure shellac dry solids having a molecular weight of approx. 1000 with water in the presence of a water-soluble film-forming agent in the range of 0.1 to 3 wt% and a gel-forming agent that is stable in the acidic range in a concentration range of 0.1 to 1 wt% and an acid for adjusting the pH to a value of 2 to 4.2 while stirring at 150 to 1000 rpm for a period of 5 to 60 minutes at 5 °C to 20 °C;
and
c) heating the emulsion or the gel from step a) to a temperature in the range of 35 °C to 45 °C while stirring and
d) adding the shellac solution or dispersion from step b) while stirring at 150 to 1000 rpm for a period of 5 to 60 minutes at 35 °C to 45 °C;
and then
e) cooling the resulting product to ambient temperature.

14. A method according to claim 13, wherein the temperature is in the range of 5 °C to 15 °C.

15. A method according to claim 13, wherein the pH is in the range of 2 to 3.5.

## Revendications

1. Produit cosmétique contenant de la shellac, **caractérisé par** une teneur en shellac à la phase aqueuse, la proportion de shellac pure dans une émulsion huile/eau étant comprise entre 0,1 et 20 % en poids, dans une émulsion eau/huile étant comprise entre 0,1 et 15 % en poids,
dans un hydrogel étant comprise entre 0,1 et 10 % en poids,
la phase aqueuse contenant en outre un agent filmogène hydrosoluble,
et un agent gélifiant constant dans un domaine acide, et la phase huileuse ou la phase aqueuse ou les deux phases contenant d'autres substances cosmétiques actives ou porteurs cosmétiques en une quantité comprise entre 1 et 20 % en poids, toutes les valeurs étant données en fonction du poids de la composition totale, et dans lequel la shellac provient d'une solution ou d'une dispersion aqueuse de shellac qui contient 5 à 60 % de shellac, 0,1 à 3 % en poids de l'agent filmogène, 0,1 à 1 % en poids de l'agent gélifiant, un acide pour établir le pH entre 2 et 4,2 et de l'eau, par rapport à la concentration totale de la solution de shellac.

2. Produit cosmétique contenant de la shellac, **caractérisé par** une teneur en shellac à la phase aqueuse, la proportion de shellac pure étant comprise,
dans une émulsion huile/eau entre 0,1 et 20 % en poids,
dans une émulsion eau/huile entre 0,1 et 15 % en poids,
dans un hydrogel entre 0,1 et 10 % en poids,
et la phase huileuse ou la phase aqueuse ou les deux phases contenant d'autres substances cosmétiques actives ou porteurs cosmétiques en une quantité comprise entre 1 et 20 % en poids,
toutes les valeurs étant données en fonction du poids de la composition totale, pouvant être obtenu
a) par fabrication d'une émulsion ou d'un hydrogel de manière habituelle par mélange d'une phase d'émulsion primaire, contenant les substances actives et les additifs cosmétiques prévus, avec une phase d'émulsion secondaire, contenant les substances actives et les additifs cosmétiques prévus,
ou mélange d'un agent gélifiant avec de l'eau et ajout des substances actives et des additifs prévus
et
b) fabrication d'une solution ou d'une dispersion aqueuses de shellac à 5 à 60 % en poids par mélange de substance sèche pure de shellac d'un poids moléculaire d'environ 1 000 avec de l'eau en présence d'un agent filmogène hydrosoluble en une quantité comprise entre 0,1 et 3 % en poids et d'un agent gélifiant constant dans un domaine de pH acide en une quantité de 0,1 à 1 % en poids et d'un acide pour établir le pH à une valeur comprise entre 2 et 4,2 sous agitation à 150 à 1 000 U/min pendant 5 à 60 minutes à une température de 5 à 20 °C
et
c) chauffage de l'émulsion ou du gel de l'étape a) à une température comprise entre 35 et 45°C sous agitation et
d) ajout de la solution ou dispersion de shellac de l'étape b) sous agitation à 150 à 1 000 U/min pendant 5 à 60 minutes à une température comprise entre 35 et 45 °C ;
et ensuite
e) refroidissement du produit obtenu à température ambiante.

3. Produit selon la revendication 2 **caractérisé en ce que** la concentration de la solution ou de la dispersion aqueuse de shellac est comprise entre 10 et 60 % en poids.

4. Produit selon la revendication 1 ou 2 **caractérisé en ce que** la concentration de la solution ou de la dispersion aqueuse de shellac est comprise entre 15 et 60 % en poids.

5. Produit selon la revendication 4 **caractérisé en ce que** la concentration de la solution ou de la dispersion aqueuse de shellac est comprise entre 15 et 50 % en poids.

6. Produit selon la revendication 1 **caractérisé en ce qu'**il s'agit d'une émulsion multiple de type eau/huile/eau ou huile/eau/huile.

7. Produit selon la revendication 1 ou 2 **caractérisé en ce que** la proportion de shellac dans le produit cosmétique fini est comprise, dans une émulsion huile/eau, entre 5 et 20 % en poids, dans une émulsion eau/huile, entre 5 et 15 % en poids, dans un hydrogel, entre 3 et 10 % en poids.

8. Produit selon la revendication 1 ou 2 **caractérisé en ce que** la proportion de shellac dans l'émulsion ou l'hydrogel est comprise entre 0,5 et 8 % en poids.

9. Produit selon l'une des revendications 1 à 8 **caractérisé en ce que** la viscosité de la solution ou de la dispersion de shellac est comprise entre 500 et 100 000 mPa.s (cP).

10. Produit selon la revendication 9 **caractérisé en ce que** la viscosité de la solution ou de la dispersion de shellac est comprise entre 1 000 et 100 000 mPa.s (cP).

11. Produit selon la revendication 9 **caractérisé en ce que** la viscosité de la solution ou la dispersion de shellac est comprise entre 2 500 et 100 000 mPa.s (cP).

12. Produit selon l'une des revendications 1 à 11 **caractérisé en ce qu'**il se présente sous forme de crème, de lotion, de préparation solaire, de stick pour les lèvres, de maquillage, de masque, de brillant pour les lèvres, de spray ou de gel.

13. Procédé pour la fabrication d'un produit cosmétique contenant de la shellac **caractérisé en ce que** les étapes suivantes sont mises en oeuvre :
a) fabrication d'une émulsion ou d'un hydrogel de manière habituelle par mélange d'une phase d'émulsion primaire, contenant les substances actives et les additifs cosmétiques prévus, avec une phase d'émulsion secondaire, contenant les substances actives et les additifs cosmétiques prévus,
ou mélange d'un agent gélifiant avec de l'eau et ajout des substances actives et des additifs prévus
et
b) fabrication d'une solution ou d'une dispersion aqueuses de shellac à 5 à 60 % en poids par mélange de substance sèche pure de shellac d'un poids moléculaire d'environ 1 000 avec de l'eau en présence d'un agent filmogène hydrosoluble en une quantité comprise entre 0,1 et 3 % en poids et d'un agent gélifiant constant dans un domaine de pH acide en une quantité de 0,1 à 1 % en poids et d'un acide pour établir le pH à une valeur comprise entre 2 et 4,2 sous agitation à 150 à 1 000 U/min pendant 5 à 60 minutes à une température de 5 à 20 °C ;
et
c) chauffage de l'émulsion ou du gel de l'étape a) à une température comprise entre 35 et 45°C sous agitation et
d) ajout de la solution ou dispersion de shellac de l'étape b) sous agitation à 150 à 1 000 U/min pendant 5 à 60 minutes à une température comprise entre 35 et 45 °C ;
et ensuite
e) refroidissement du produit obtenu à température ambiante.

14. Procédé selon la revendication 13 **caractérisé en ce que** la température est comprise entre 5 et 15 °C.

15. Procédé selon la revendication 13 **caractérisé en ce que** la valeur du pH est comprise entre 2 et 3,5.
